# EUROPÄISCHE PATENTSCHRIFT

(11) **EP 1 087 794 B1**
(45) Veröffentlichungstag und Bekanntmachung des Hinweises auf die Patenterteilung: **07.12.2005**
(21) Anmeldenummer: 99929276.6
(22) Anmeldetag: 18.06.1999
(51) Int. Cl.: A61K 49/00, A61K 41/00, A61K 31/195, A61C 17/02, A61C 19/06, A61B 5/00, A61N 5/06

(54) **LICHTAPPLIKATIONSEINRICHTUNG ZUR PHOTODYNAMISCHEN DIAGNOSE UND/ODER PHOTODYNAMISCHEN THERAPIE VON ERKRANKUNGEN DES ZAHNHALTEAPPARATES UND DER ZÄHNE**
LIGHT APPLICATION DEVICE FOR PHOTODYNAMIC DIAGNOSIS OR PHOTODYNAMIC THERAPY OF DISEASES OF THE PARODONTIUM AND TEETH
DISPOSITIF D'APPLICATION DE LUMIERE POUR LE DIAGNOSTIC OU LA THERAPIE PHOTODYNAMIQUES D'AFFECTIONS DU PARODONTE ET DES DENTS

(30) Priorität: 19.06.1998 DE 19827417
(43) Veröffentlichungstag der Anmeldung: 04.04.2001
(73) Patentinhaber: Karl Storz GmbH & Co. KG, 78532 Tuttlingen (DE)
(72) Erfinder: IRION, Klaus, M., D-78576 Liptingen (DE)
(74) Vertreter: Weller, Wolfgang
(86) Internationale Anmeldenummer: PCT/EP1999/004242
(87) Internationale Veröffentlichungsnummer: WO 1999/066831

(56) Entgegenhaltungen:
- EP-A- 0 300 277
- WO-A-94/17797
- WO-A-95/07077
- WO-A-95/10243
- WO-A-96/39188
- WO-A-98/06456
- WO-A-98/09155
- WO-A-98/30242
- DE-A- 2 725 793
- DE-U- 8 517 634
- DE-U- 29 705 934
- FR-A- 1 171 206
- US-A- 4 685 596
- US-A- 5 033 961
- US-A- 5 098 291
- US-A- 5 422 093
- US-A- 5 558 518
- US-A- 5 620 700
- LEUNIG, ANDREAS ET AL: "Fluorescence imaging and spectroscopy of 5- aminolevulinic acid induced protoporphyrin IX for the detection of neoplastic lesions in the oral cavity" AM. J. SURG., 1996, VOL. 172, NO. 6, PAGE(S) 674-677, XP002120195
- LEUNIG A. ET AL: "PHOTODYNAMISCHE DIAGNOSTIK VON NEOPLASIEN DER MUNDHÖHLE NACH LOKALER APPLIKATION VON 5-AMINOLÄVULINSÄURE" LARYNGO- RHINO- OTOLOGIE, 1996, VOL. 75, NO. 8, PAGE(S) 459-464, XP002120196
- VONARX V ET AL: "Potential efficacy of a delta 5-aminolevulinic acid bioadhesive gel formulation for the photodynamic treatment of lesions of the gastrointestinal tract in mice." J PHARM PHARMACOL, JUL 1997, VOL. 49, NO. 7, PAGE(S) 652-6, XP002078605 ISSN: 0022-3573
- MESSMANN H ET AL: "Photodynamische Diagnostik gastrointestinaler Prakanzerosen nach Sensibilisierung mit 5-Aminolavulinsaure. Eine Pilotstudie." DTSCH MED WOCHENSCHR, 24-4-1998, VOL. 123, NO. 17, PAGE(S) 515-21, XP002120197
- ROPER J.M. ET AL: "Tetrapyrrole biosynthesis in several haem-dependent anaerobic pathogens" REVIEWS IN MEDICAL MICROBIOLOGY, 1997, VOL. 8, NO. SUPPL. 1, PAGE(S) S13-S17, XP002120198
- BAUMGARTNER R ET AL: "Inhalation of 5-aminolevulinic acid: a new technique for fluorescence detection of early stage lung cancer." J PHOTOCHEM PHOTOBIOL B, NOV 1996, VOL. 36, NO. 2, PAGE(S) 169-74, XP002120199 ISSN: 1011-1344
- WEBBER J ET AL: "On-line fluorescence of human tissues after oral administration of 5-aminolevulinic acid." J PHOTOCHEM PHOTOBIOL B, APR 1997, VOL. 38, NO. 2-3, PAGE(S) 209-14, XP002120200 ISSN: 1011-1344
- PENG Q ET AL: "5-Aminolevulinic acid-based photodynamic therapy. Clinical research and future challenges." CANCER, JUN 15 1997, VOL. 79, NO. 12, PAGE(S) 2282-308, XP002120201 ISSN: 0008-543X
- ACKERMANN G ET AL: "Simulations on the selectivity of 5-aminolaevulinic acid-induced fluorescence in vivo." J PHOTOCHEM PHOTOBIOL B, DEC 1998, VOL. 47, NO. 2-3, PAGE(S) 121-8, XP002120202 ISSN: 1011-1344

## Beschreibung

Die Erfindung betrifft eine Lichtapplikationseinheit zur kombinierten photodynamischen Diagnose und/oder photodynamischen Therapie von nicht-malignen Erkrankungen des Zahnhalteapparates und der Zähne, mit einer Lichtquelle, die zumindest Licht im sichtbaren Bereich erzeugt, mit einer Fokussiereinheit zum Fokussieren des Lichtes, mit zumindest einem Lichtleiter, der Licht von der Lichtquelle zu einem distalen, abstrahlenden Ende der Lichtleiter führt, mit zumindest einem im Strahlengang des Lichts angeordneten Element, mit dem die spektralen Eigenschaften des Lichtes der Lichtquelle veränderbar sind, wobei der Lichtleiter zumindest in einem distalen Handhabungsbereich rigide ausgebildet ist und zwei Lichtleiter vorgesehen sind.

Eine Lichtapplikationseinheit mit diesen konstruktiven Merkmalen ist aus der DE-A-196 39 653 bekannt.

Eine Verwendung von 5-Aminolävulinsäure für die photodynamische Diagnose (PDD) zur Früherkennung eines Harnblasenkarzinoms ist aus dem Sonderdruck "Endo World" URO Nr. 17/1-D, 1997 der KARL STORZ GmbH & Co., Tuttlingen, Deutschland und der KARL STORZ ENDOSCOPY, Amerika bekannt.

Daraus ist bekannt, daß bestimmte Photosensitizer in Verbindung mit speziellem Licht dazu verwendet werden können, malignes oder anderweitig entartetes Gewebe zu erkennen (photodynamische Diagnose, PDD) und zu zerstören (photodynamische Therapie, PDT). Das Phänomen wird in malignem Gewebe beobachtet, gesundes Gewebe zeigt dieses Phänomen nicht.

Dieses Phänomen beruht auf der Gabe von 5-Aminolävulinsäure und einer dadurch induzierten Bildung von photosensiblem Protoporphyrin IX (PPIX). 5-Aminolävulinsäure ist ein Prekursor für den Photosensitizer Protoporphyrin IX, so daß durch Verabreichung dieses Prekursors die Menge an Protoporphyrin IX erhöht werden kann. Von gesundem Gewebe wird der Prekursor nicht angereichert und in PPIX umgesetzt.

Der Prekursor für den Photosensitizer wird durch Installation, Spülung, Inhalation, oral oder topisch appliziert. Mit einem entsprechenden Lichtapplikationssystem kann dieses Gewebe zur Fluoreszenz angeregt und diese Fluoreszenz beobachtet werden, wodurch dann die photodynamische Diagnose (PDD) eröffnet ist. Ferner wurde festgestellt, daß ein PPIX-induzierter phototoxischer Effekt auftritt, der somit die photodynamische Therapie (PDT) eröffnet.

Aus der US-A-5 422 093 ist ein Verfahren zum Detektieren und Behandeln von malignen und nicht-malignen Gewebeabnormalitäten bekannt. Dabei wird 5-Aminolävulinsäure oder ein Prekursor davon verabreicht, um in den abnormalen Zellen eine Anreicherung von Protoporphyrin IX zu erzielen, was durch Fluoreszenz detektiert werden kann.

Aus der WO-A-95/07077 ist eine pharmazeutische zusammensetzung zur Behandlung von Abnormalitäten von äußeren oder innenliegenden Körperoberflächen bekannt, die auf eine Photochemotherapie ansprechen, wobei 5-Aminolävulinsäure oder ein Prekursor davon zusammen mit einem Agens verabreicht wird, das die Penetration durch die Oberfläche fördert.

Aus der WO-A-98/06456 ist eine Vorrichtung zum Durchführen einer Lichttherapie im Inneren einer Mundhöhle bekannt. Ein etwa U-förmiger Abschnitt kann über die Zahnreihe und das Zahnfleisch gelegt werden. Eine Lichtquelle erzeugt Licht mit bestimmter Wellenlänge, die von einem photoreaktiven Agens, das zuvor dem Zahnfleisch verabreicht wurde, absorbiert wird. Dadurch können krankhafte Gewebe und unerwünschte Organismen entfernt werden.

Aus der WO-A-96/28412 sind Ester von 5-Aminolävulinsäure beschrieben, die als Photosensitizer in der photodynamischen Therapie eingesetzt werden können.

Aus der EP-A-0 709 698 ist ein Lichtleiter bekannt, der aus einer Vielzahl von optischen Fasern zusammengesetzt ist, die miteinander gebündelt sind. Zur Lichtkonzentration ist vorgesehen, in einem ersten, sich konisch verjüngenden Abschnitt die Bündel enger aneinander zu führen, wobei dieser in einen distal gekrümmten Abschnitt übergeht.

Aufgabe der Erfindung ist es, eine Lichtapplikationseinheit zur kombinierten photodynamischen Diagnose und/oder photodynamischen Therapie von nicht-malignen Erkrankungen des Zahnhalteapparates und der Zähne zu schaffen.

Erfindungsgemäß wird die Aufgabe dadurch gelöst, daß bei der Lichtapplikationseinheit die Lichtleiter distalseitig einen gekrümmten Abschnitt aufweisen, und daß die beiden Lichtleiter nebeneinander verlaufen, wobei eine abstrahlseitige Stirnfläche des einen Lichtleiters über die abstrahlseitige Stirnfläche des anderen Lichtleiters distalseitig hinausragt.

Dentale Erkrankungen, wie Karies oder Parodontopathien, gehören zu den am weistest verbreiteten Erkrankungen.

Derzeitige Behandlungsmethoden der Parodontitis basieren zum einen auf einer instrumentengestützten, rein mechanischen oder ultraschallgestützten Reinigung des Zahnfleisches oder der Zahnfleischränder bzw. der Zahnoberfläche und der Zahnfleischtaschen, zum anderen auf einer Spülung des entzündeten Gewebes mit antibakteriellen chemischen Substanzen, mit dem Ziel, die Bakterien, welche die Entzündung verursachen, zu zerstören.

Befallene und nichtbefallene Gewebeareale sind dabei visuell zum Teil nur schwer zu differenzieren. Bei schwergradigen Entzündungen und bei rasch verlaufenden Paradontopathien ist nur eine hochdosierte systemische Antibiotikagabe zur Eindämmung der Erkrankung möglich, da im parodontalen weichgewebe tieferliegende Bakterien über mechanische bzw. ultraschallgestützte Reinigung und Spülungen nicht oder nur zum Teil inaktiviert werden können. Eine derartige Antibiotika-Behandlung weist jedoch eine Vielzahl von Nebenwirkungen auf, z.B. Zerstörung der Darmflora oder Resistenzbildung, so daß diese Therapie nicht zufriedenstellend ist.

Karies wird ebenfalls durch Bakterien ausgelöst, meist als Folge einer Zahndemineralisation durch Monosaccharide.

Es konnte nun überraschenderweise festgestellt werden, daß unter Verwendung von 5-Aminolävulinsäure oder Derivaten davon erfolgreich sowohl eine photodynamische Diagnose als auch eine photodynamische Therapie von nicht-malignen Erkrankungen des Zahnhalteapparates und der Zähne durchgeführt werden kann. Dies ist insoweit überraschend, als eine ganze Reihe an sich unterschiedlicher Erreger oder Keime für Erkrankungen des Zahnhalteapparates oder der Zähne ursächlich sind, dennoch unter Verwendung von 5-Aminolävulinsäure oder Derivaten davon eine aussagekräftige photodynamische Diagnose durchgeführt werden kann und, was noch wesentlich bedeutsamer ist, eine erfolgreiche photodynamische Therapie durchgeführt werden kann.

Durch die Ausgestaltung der Lichtapplikationseinheit mit den eingangs genannten Merkmalen kann über den distalseitig gekrümmten Abschnitt des Lichtleiters das Licht gezielt an den Zahnhalteapparat bzw. an die Zähne herangeführt werden, insbesondere auch in die bei derartigen Erkrankungen des Zahnhalteapparates entstehenden Zahnfleischtaschen zwischen Zahnfleisch und Zahn.

Die 5-Aminolävulinsäure bzw. deren Derivate können, wie bekannt, z.B. oral, parenteral, systemisch, jedoch auch topisch appliziert werden, wobei die entstandenen Zahnfleischtaschen dazu herangezogen werden können, darin das pharmazeutische Präparat einzubringen, und zwar derart, daß dieses dort verbleibt, so daß die 5-Aminolävulinsäure bzw. deren Derivat dann in die entsprechenden Gewebebereiche eindringen kann. Dazu weist die Vorrichtung zum Applizieren eine für diesen Einsatzzweck geeignete Kanüle auf.

Die 5-Aminolävulinsäure oder eines ihrer Derivate werden zur Herstellung eines pharmazeutischen Präparates zur photodynamischen Diagnose und/oder photodynamischen Therapie von Parodontitis, Parodontopathien, Karies, von oberflächlich auf dem Gewebe des Zahnhalteapparats befindlichen Bakterien oder von im Gewebe des Zahnhalteapparates befindlichen Bakterien herangezogen.

Diese Krankheitsbilder sind die am häufigsten auftretenden Krankheitsbilder von Erkrankungen des Zahnhalteapparates bzw. der Zähne, die allesamt durch Anwendung von 5-Aminolävulinsäure bzw. eines ihrer Derivate sowohl diagnostiziert als auch therapiert werden können.

Parodontopathien sind entzündliche (> 90 %) degenerative (bis 4 %) und hyperplastische (ca. 1 %) Erkrankungen des marginalen Zahnhalteapparates multifaktorieller Ätiologie.

Unter Paradontitis versteht man eine Entzündung des Zahnhalteapparates. Unter Parodontose wird eine degenerative Form der Parodontopathie mit Schwund des marginalen Zahnhalteapparates aufgrund primär-regressiver, nicht-entzündlicher Prozesse mit Taschenbildung und Zahnlockerung verstanden.

Je nach Fortschreiten und Art des Befalles mit Bakterien befinden sich diese lediglich oberflächlich auf dem Gewebe des Zahnhalteapparates oder sind in das Gewebe eingedrungen.

Die nun mögliche photodynamische Diagnose erlaubt in einem ersten Schritt eine Lokalisierung der befallenen Stellen und eröffnet somit dann die Möglichkeit, anschließend in einer gezielten photodynamischen Therapie diese Stellen zu behandeln.

Die 5-Aminolävulinsäure oder eines ihrer Derivate werden in einer Trägersubstanz verwendet, ausgewählt aus der Gruppe bestehend aus Hydrogelen, insbesondere Alginatgel, einer Öl-in-Wasser-Emulsion oder einer Pufferlösung, die einen pH 5-6 einstellt.

Diese Stoffe sind zum einen äußerst verträglich und erlauben eine topische Anwendung derart, daß der Prekursor für den Photosensitizer durch die Trägersubstanz ausreichend lange an Ort und Stelle gehalten wird, so daß dieser dann in das Gewebe bzw. in die befallenen Stellen hineindiffundieren kann. Dieser Vorgang benötigt gegebenenfalls mehrere Stunden, so daß die Gefahr besteht, daß durch den Speichel in der Mundhöhle der Prekursor weggespült wird. Die Trägersubstanz in Form der zuvor genannten Ausgestaltungen verhindert ein Aus- oder Abspülen durch den Speichel. Es besteht auch die Möglichkeit einer oralen Gabe, oder systemischen Gaben, was keine lokale Applikation des Prekursors mit Träger notwendig macht.

Als Derivat wird ein Ester der 5-Aminolävulinsäure eingesetzt. Die Verwendung von Estern hat zum einen den Vorteil, daß derartige Ester chemisch stabiler sind als die 5-Aminolävulinsäure selbst. Es ist zu bedenken, daß in der Mundhöhle nicht nur Speichel sondern auch unterschiedliche Enzyme ständig vorhanden sind, die zu raschen Zersetzungsreaktionen der 5-Aminolävulinsäure führen könnten. Ein weiterer Vorteil der Ester besteht darin, daß diese eine wesentlich höhere Gewebepenetration aufgrund einer besseren Lipophylität als 5-Aminolävulinsäure aufweisen. Dies eröffnet nunmehr im Bereich der Zahnheilkunde die Möglichkeit, möglichst rasch nach Applizieren des Prekursors des Photosensitizers eine Diagnose und/oder eine Therapie durchzuführen. Beispielsweise kann der Patient nach Applizieren schon nach einer relativ geringen Wartezeit diagnostiziert werden. Aufgrund der schnelleren Penetration können auch wesentlich geringere Konzentrationen an Estern eingesetzt werden, so daß schon dadurch mögliche Nebenwirkungen reduziert oder unterdrückt werden können. Müssen beispielsweise bei der Säure Konzentrationen von 160-200 mMol eingesetzt werden, so reicht vergleichsweise beim Hexylester eine Konzentration von 4-16 mMol.

Ein noch wesentlicher, weiterer Vorteil der Ester, insbesondere des Methylesters, des Ethylesters und, besonders signifikant, des Hexylesters, besteht darin, daß diese eine wesentlich stärkere und auch homogenere Fluoreszenz verursachen. So verursacht beispielsweise der Hexylester von 5-Aminolävulinsäure eine gegenüber der Säure um 50fach stärkere Fluoreszenz. Der Hexylester weist auch eine etwa um den Faktor 2 höhere Gewebepenetration gegenüber der Säure auf. Dadurch ist nun auf dem speziellen Gebiet der Zahnheilkunde der Weg eröffnet, daß der Patient in die Praxis kommt, das pharmazeutische Präparat, das den Prekursor für den Photosensitizer enthält, appliziert bekommt, und schon nach einer kurzen Wartezeit die Diagnose erstellt oder eine Therapie begonnen werden kann. Dies erleichtert auch erheblich die praktische Umsetzung bzw. die Akzeptanz beim Patienten, die sogenannte Compliance.

Einer der beiden Lichtleiter kann in eine Zahntasche zwischen Zahn und Zahnfleischgewebe eingeschoben werden kann und ein weiterer von der Außenseite her an das Zahnfleisch herangebracht werden kann. Dadurch kann gezielt und von zwei Seiten her das Anregungslicht auf einen befallenen Bereich des Zahnfleisches gebracht werden. Insbesondere, wenn die Bakterien schon tief in das Zahnfleisch eingedrungen sind, kann durch die beidseitige Bestrahlung der befallene Bereich optimal, insbesondere bei der photodynamischen Therapie, bestrahlt werden, so daß schon nach kurzen intensiven Bestrahlungszeiten erfolgreich therapiert werden kann.

Bei der Erfindung laufen die beiden Lichtleiter nebeneinander, wobei eine abstrahlseitige Stirnfläche des einen Lichtleiters über die abstrahlseitige Stirnfläche des anderen distalseitig hinausragt.

Diese Ausgestaltung hat den Vorteil, daß der eine, weiter vorragende Lichtleiter beispielsweise zunächst gezielt in eine Tasche zwischen Zahnfleisch und Zahn eingeschoben werden und dann der andere von der Außenseite an das Zahnfleisch herangelegt werden kann, oder, wenn die Tasche nicht tief ist, genau umgekehrt der weiter vorragende über einen großen Flächenbereich an die Außenseite des Zahnfleisches herangelegt werden kann und der kürzere nur in die relativ kleine Tasche eingeschoben werden kann.

In einer weiteren Ausgestaltung der Erfindung ist ein Lichtleiter axial relativ zum anderen Lichtleiter verschiebbar.

Diese Maßnahme hat den Vorteil, daß die zuvor schon erwähnte Handhabung nun noch in ihrer Variabilität erhöht ist, was dem Zahnarzt eine optimale Anpassung an das jeweilige Krankheitsbild und eine optimale Anpassung an die Geometrie des Zahnhalteapparates des Patienten ermöglicht, insbesondere bei Anomalien und problematischen Stellungen der Zähne.

In einer weiteren Ausgestaltung der Erfindung ist der verschiebliche Lichtleiter in einem rohrförmigen Kanal geführt.

Diese Maßnahme hat den Vorteil, daß der verschiebliche und gegebenenfalls relativ dünne Lichtleiter über eine lange Strecke exakt geführt ist, dessen Bewegungen also sicher gesteuert werden.

In einer weiteren Ausgestaltung der Erfindung ist der rohrförmige Kanal zur Führung von Medien ausgebildet.

Diese Maßnahme hat nun den erheblichen Vorteil, daß durch den Kanal therapieunterstützend gespült oder gesaugt bzw. ein Gas insuffliert werden kann. Gekühlte Druckluft trägt zur subjektiven Schmerzlinderung bei der PDT bei. Zugeführter Sauerstoff sorgt für eine Beschleunigung der PDT-Wirkung. Über diesen Kanal ist es auch möglich, das pharmazeutische Präparat, das den Prekursor enthält, zu applizieren.

In einer weiteren Ausgestaltung der Erfindung ist das im Strahlengang anordenbare Element als Filter ausgebildet. Dabei sind insbesondere unterschiedliche Filter in den Strahlengang ein- bzw. ausbringbar.

Eines der Filter transmittiert im Bereich des Anregungsspektrums des verwendeten Photosensitizers, dient also zur Fluoreszenzanregung im entzündeten Gewebe bei der photodynamischen Diagnose. Transmittiert das zweite Filter zumindest im Bereich der Fluoreszenzabstrahlung, so dient dies der photodynamischen Therapie des Gewebes, speziell der entzündeten Gewebebereiche.

In einer weiteren Ausgestaltung der Erfindung ist ein drittes Filter vorgesehen, das in die Blickrichtung von einer Handhabungsperson zu einer Behandlungsstelle am Zahnhalteapparat bringbar ist, das Fluoreszenzanregungslicht abblockt, jedoch Fluoreszenstrahlung durchläßt.

Diese Maßnahme hat den Vorteil, daß von einer Handhabungsperson die gewebecharakterisierende Fluoreszenz einfach detektiert werden kann.

In einer weiteren Ausgestaltung der Erfindung ist ein Lichtleiter aus einer Vielzahl von lichtleitenden Einzelfasern zusammengesetzt.

Diese Maßnahme hat den Vorteil, daß dadurch das Licht problemlos über die Krümmung hinweg transportiert werden kann. Der aktive Durchmesser der Einzelfaser liegt dabei im Bereich von 20 bis 400 *µ*m. Durch Verschmelzung der Einzelfaser zu einer Art Glasstab entsteht ein ausreichend rigides Gebilde, so daß keine weitere zusätzliche Ummantelung erforderlich ist. Dadurch ist auch eine ausreichende Biokompatibilität und Sterilisierfähigkeit z.B. im Autoklaven gegeben.

In einer weiteren Ausgestaltung der Erfindung besteht der Lichtleiter aus einer einzigen lichtleitenden Faser.

Diese Maßnahme hat herstellungstechnische Vorteile. Im Handhabungsbereich muß gegebenenfalls dieser Lichtleiter durch ein Umhüllungsrohr verstärkt werden, um die ausreichende Steifigkeit zu erlangen.

Die einzige Faser besitzt einen aktiven Durchmesser im Bereich von etwa 200 bis 2.000 *µ*m. Dieser kleinkalibrige Lichtleiter besitzt den Vorteil, daß direkt in der Zahnfleischtasche bestrahlt werden kann.

Die Lichtleitfaser kann sowohl ein Glas-, Kunststoff-, Quarzlichtleiter oder auch ein Flüssiglichtleiter sein.

In einer weiteren Ausgestaltung der Erfindung ist ein weiterer Lichtleiter vorgesehen, der Licht von der bestrahlten Stelle nach proximal leitet. Dieser weitere Lichtleiter kann Teil eines mehrfaserigen Lichtleiters ein, der an sich Licht nach distal leitet.

Diese Maßnahmen haben den Vorteil, daß das rückgeführte Licht zur Detektion der Fluoreszenz und somit auch zur Dosimetrie bei der PDT herangezogen werden kann. So kann die Bestimmung des Ausbleichens des Photosensitizers über den PDD-Modus zur Dosimetrie bzw. zur zeitlichen Kontrolle des PDT-Vorganges herangezogen werden.

Je nach Ausgestaltung des Lichtleiters kann nicht nur Licht, sondern, durch geordnete Einzelfasern, auch ein ganzes Bild zurückgeleitet werden, das dann einem Bilderfassungs- und/oder Bildverarbeitungssystem z.B. mit einer miniaturisierten CCD-Kamera zugeführt werden kann.

In einer weiteren Ausgestaltung der Erfindung wird das vom weiteren Lichtleiter nach proximal geleitete Licht einer Auswerteeinheit zugeführt, wobei die Auswerteeinheit vorzugsweise eine Spektralanalyseeinheit, eine Kamera oder ein spektral selektiv sensitives Photoelement aufweist.

Diese Maßnahmen haben den Vorteil, daß das vom weiteren Lichtleiter zurückgeführte Licht bzw. Bild über eine entsprechende Auswerteinheit erfaßt, quantifiziert und beispielsweise dadurch die örtliche Information des Ausbleichvorganges verfolgt werden kann und somit die photodynamische Behandlung kontrolliert und berechnet werden kann.

In einer weiteren Ausgestaltung der Erfindung ist der Lichtleiter distalseitig mit einem Abstandhalter versehen.

Diese Maßnahme hat den Vorteil, daß dadurch eine konstante Bestrahlungsintensität erreicht werden kann, somit eine bessere Dosimetrie bei der photodynamischen Therapie.

In einer weiteren Ausgestaltung der Erfindung weist der Lichtleiter distalseitig einen Diffusor auf. Dabei kann der Diffusor als kugelförmig konvexe Aufweitung des distalen Endes des Lichtleiters ausgebildet sein, oder der Diffusor kann als lichtdurchlässiges Material mit optisch streuenden Eigenschaften ausgebildet sein, oder der Diffusor kann als füllbarer Ballon ausgebildet werden, der am distalen Ende des Lichtleiters angeordnet ist und über einen Kanal befüllbar ist.

Diese Maßnahmen haben den Vorteil, daß durch den Diffusor das an sich relativ gebündelt und gezielt an dem relativ kleinen stirnseitigen Austrittsende des Lichtleiters austretende Licht gleichmäßig verteilt über einen großen Flächenbereich abgestrahlt werden kann, so daß dann gleichmäßige Behandlungsbedingungen beispielsweise in der gesamten Zahnfleischtasche vorherrschen, in der ein solcher Lichtleiter eingeschoben ist.

In einer weiteren Ausgestaltung der Erfindung ist ein Ultraschallübertragungselement vorgesehen, über das Ultraschall zum distalen Ende leitbar ist.

Diese Maßnahme hat den Vorteil, daß die parallele Applikation von Ultraschall (z.B. 20 KHz bis 3 MHz) zu einer Steigerung der Wirkung bei der photodynamischen Therapie führt und somit zu einer schnelleren Inaktivierung von Bakterien. Es hat sich gezeigt, daß durch Ultraschalleinwirkung die Penetrationstiefe des Prekursors des Photosensitizers erhöht wird und sich somit die gewünschte Tiefenwirkung der Behandlung verbessert.

In einer weiteren Ausgestaltung der Erfindung ist eine Ultraschallanregungseinheit vorgesehen, die mit dem Ultraschallelement gekoppelt ist.

Diese Maßnahme hat den Vorteil, daß die Lichtapplikationseinheit selbst auch zur Ultraschallerzeugung ausgelegt ist.

In einer weiteren Ausgestaltung der Erfindung ist ein Lichtleiter als Ultraschallübertragungselement ausgebildet.

Diese Maßnahme hat den Vorteil, daß durch baulich einfache Maßnahmen sowohl das Licht als auch der Ultraschall geleitet wird, nämlich durch ein und dasselbe Element, nämlich den Lichtleiter. Dazu kann beispielsweise ein piezoelektrischer Antrieb an den Lichtleiter, insbesondere an einen Quarzlichtleiter, angelegt werden.

In einer weiteren Ausgestaltung der Erfindung weist die Lichtquelle einen hohen Blauanteil im Bereich von etwa 400 nm (± 20 nm) auf.

Diese Maßnahme hat den Vorteil, daß man im Fluoreszenzanregungsbereich des von dem Prekursor erzeugten Protoporphyrin IX liegt, somit speziell darauf ausgerichtet ist.

In einer weiteren Ausgestaltung der Erfindung wird als Lichtquelle eine Xenon-Kurzbogenlampe eingesetzt.

Diese Maßnahme hat den Vorteil, daß eine Lichtquelle mit einem hohen Blauanteil, einer hohen Bestrahlungsstärke und mit einem relativ kleinen Brennfleck zur Verfügung steht. Dadurch ist bei der Therapie eine hohe Lichteindringtiefe möglich.

In einer weiteren Ausgestaltung der Erfindung weist die Lichtapplikationseinheit ein Gehäuse mit einem pistolenartigen Handgriff auf, von dem ein Rohrabschnitt vorsteht, in dem die Lichtleiter geführt sind.

Diese Maßnahme hat den Vorteil, daß eine Einhandbedienung auf ergonomische Art und Weise möglich ist.

In einer weiteren Ausgestaltung der Erfindung ist der Rohrabschnitt an das Gehäuse ankoppelbar bzw. von diesem abnehmbar ausgebildet.

Diese Maßnahme hat den Vorteil, daß unterschiedliche Lichtleiter, d.h. unterschiedlicher Dimension, wie Durchmesser, Länge, Krümmung, je nach Anwendungsfall einfach angekoppelt werden können.

Dabei ist weiter von Vorteil, wenn der Lichtleiter und der Rohrabschnitt zumindest in einem Abschnitt konisch verlaufen, und zwar nach distal, wodurch eine verjüngte distale Lichtleiterspitze entsteht, die besser an das Paradontium geführt werden kann. Der Konus bewirkt außerdem eine Art Lichtfokussierung und eine Erhöhung des distalseitigen Abstrahlwinkels mit der Wirkung einer homogeneren Ausleuchtung.

In einer weiteren Ausgestaltung der Erfindung ist das dritte Filter am Rohrabschnitt angebracht und ist dort vorzugsweise verschieblich und verschwenkbar angebracht.

Diese Maßnahme hat den Vorteil, daß dadurch eine einfache und flexible Positionierung des Beobachtungsfilters durchgeführt werden kann, um für die jeweilige Handhabungsposition günstige Beobachtungs- und Sichtverhältnisse zu schaffen.

In einer weiteren Ausgestaltung der Erfindung ist eine Kanüle vorgesehen, über die das pharmazeutische Präparat applizierbar ist.

Diese Maßnahme hat den Vorteil, daß die an sich nur zur Lichtapplikation vorgesehene Einheit als multifunktionales Gerät ausgebildet ist, d.h. zugleich auch zum Applizieren des pharmazeutischen Präparates herangezogen werden kann.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert.

Es zeigen:
- Fig. 1: eine stark schematisierte Seitenansicht einer erfindungsgemäßen Lichtapplikationseinheit,
- Fig. 2: eine noch stärker vereinfachte, entsprechend schematisierte Seitenansicht einer weiteren Variation mit einer Ultraschallerzeugungseinheit,
- Fig. 3: eine Variante eines abnehmbaren Rohrabschnittes der Lichtapplikationseinheit,
- Fig. 4: eine der Darstellung von Fig. 3 entsprechende Darstellung einer weiteren Variation eines Rohrabschnittes zur Applikation des pharmazeutischen Präparates, und
- Fig. 5: eine Lichtapplikationseinheit bei der Anwendung während einer photodynamischen Therapie an einem Zahnhalteapparat.

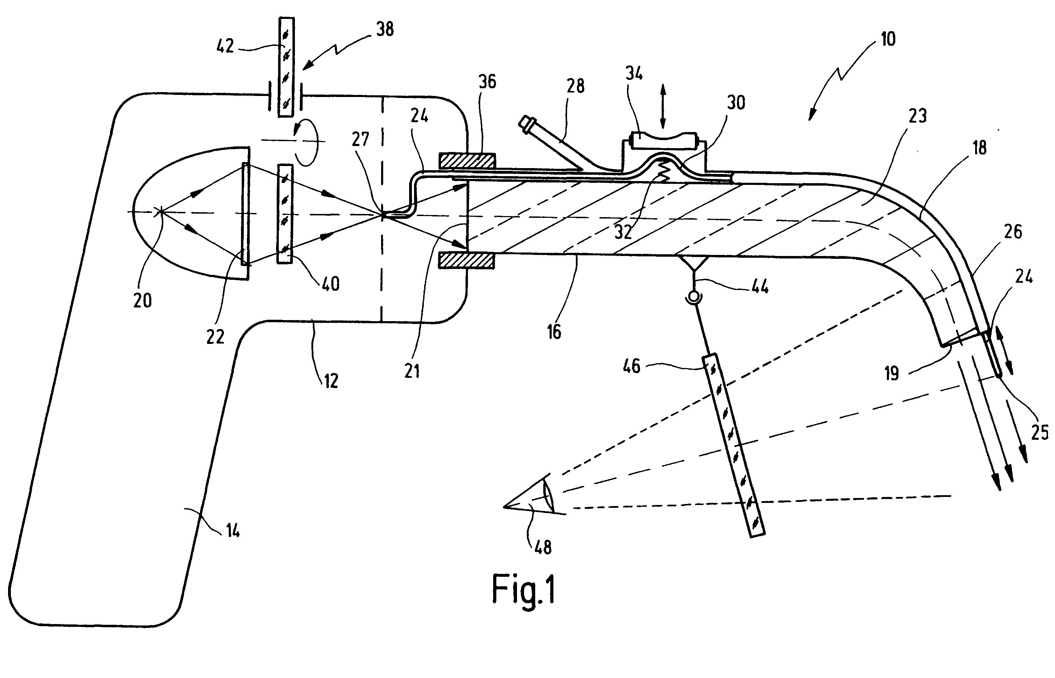

Eine in Fig. 1 dargestellte Lichtapplikationseinheit ist in ihrer Gesamtheit mit der Bezugsziffer 10 versehen.

Die Lichtapplikationseinheit 10 weist ein Gehäuse 12 auf, von dem proximalseitig ein pistolenartiger Handgriff 14 abgewinkelt absteht. An dem dem Handgriff 14 gegenüberliegenden Ende springt vom Gehäuse 12 ein Rohrabschnitt 16 vor, der distalseitig in einen gekrümmten Abschnitt 18 übergeht und der zu seinem distalen Ende 19 hin sich etwas konisch verjüngt.

Im Gehäuse 12 ist eine Lichtquelle 20 und eine Fokussiereinheit 22 aufgenommen, die das von der Lichtquelle 20 kommende Licht auf das proximale Ende 21 eines ersten Lichtleiters 23 fokussiert. Je nach Ausgestaltung der Lichtapplikationseinheit 10 wird die Lichtquelle 20 von einer im Gehäuse 12 angeordneten Energiequelle gespeist, oder steht über ein Kabel mit einer abseitigen Energiequelle in Verbindung.

Der erste Lichtleiter 23 selbst ist als gebündeltes Glasfaser aus einer Vielzahl von einzelnen Glasfasern ausgebildet, die untereinander verklebt sind. Der erste Lichtleiter 23 füllt den inneren Hohlraum des Rohrabschnittes 16 aus. Die konische Verjüngung zum distalen, Anregungslicht abstrahlenden Ende 19 hin sorgt für eine Art Fokussierung und für eine Erhöhung des Abstrahlwinkels distalseitig, was zu einer homogeneren Ausleuchtung führt.

Ein zweiter Lichtleiter 24 ist in einem Kanal 26 geführt, der im Innern des Rohrabschnitts 16 angeordnet ist. Der zweite Lichtleiter 24 kann ebenfalls aus einem Bündel an Einzelfasern zusammengesetzt sein oder eine Einzelfaser sein.

Vom Kanal 26 steht ein Anschluß 28 vor, über den der Kanal 26 mit weiteren Medien, z.B. Luftsauerstoff oder einer Flüssigkeit, versorgt werden kann. Diese Medien werden im freien Lumen zwischen dem zweiten Lichtleiter 24 und der Innenseite des Kanals 26 geführt. Ein proximales Ende 27 des zweiten Lichtleiters 24 liegt im Fokussbereich der Fokussiereinheit und dort wird diesem Lichtleiter Licht eingespeist.

Im Bereich des geradlinigen Abschnitts des Rohrabschnitts 16 ist der zweite Lichtleiter 24 in Form einer Welle 30 geführt, unter der eine Druckfeder 32 angeordnet ist. Der Berg der Welle 30 liegt an der Unterseite eines Druckknopfes 34 an, der in einem hier nicht näher bezeichneten Gehäuse geführt ist. Die gegenüberliegende Seite der Feder 32 stützt sich auf der Außenseite des Rohrabschnitts 16 ab.

Distalseitig überragt der zweite Lichtleiter 24 den Kanal 26. Vom äußeren Ende 25 des zweiten Lichtleiters 24 wird Licht abgestrahlt, das über die Fokussiereinheit 22 proximalseitig in den zweiten Lichtleiter 24 eingeführt wird. Wird auf den Druckknopf 34 gedrückt, flacht die Welle 30 etwas ab, und dabei wird der zweite Lichtleiter 24 weiter aus dem Rohrabschnitt 16 hinausgeschoben. Wird der Druckknopf 34 freigegeben, drückt die Feder 32 den Druckknopf 34 wieder nach oben, und der zweite Lichtleiter 24 wird eingezogen.

Mit der Bezugsziffer 36 ist schematisch eine Kupplung bezeichnet, über die der Rohrabschnitt 16 mit dem Gehäuse 12 gekoppelt ist.

Die Kupplung 36 besteht typischerweise aus einer Bajonettkupplung. Somit ist es durch einen einfachen Löse- bzw. Ankuppelvorgang möglich, den Rohrabschnitt 16 vom Gehäuse 12 abzunehmen bzw. diesen oder einen anderen Rohrabschnitt, wie das nachstehend noch erläutert wird, anzusetzen. Es ist auch möglich, die Kupplung als Steckkupplung oder als Schraubkupplung auszubilden.

Im Gehäuse 12 sind Elemente 38 angeordnet, die in den Strahlengang zwischen Lichtquelle 20 und die Lichtleiter 23 und 24 gebracht werden können.

Diese Elemente weisen ein erstes Filter 40 sowie ein zweites Filter 42 auf, die über einen nicht näher dargestellten Mechanismus jeweils in den Strahlengang ein- bzw. ausgeschwenkt werden können.

Das erste Filter 40 ist so ausgebildet, daß es im Bereich des Anregungsspektrums des Photosensitizers transmittiert.

Das zweite Filter 42 ist so ausgebildet, daß es zumindest im Bereich der Fluoreszenzabstrahlung des Photosensitizers transmittiert.

Im dargestellten Modus ist das erste Filter 40 eingeschwenkt, läßt im wesentlichen nur das Fluoreszenzanregungslicht durch. In dieser Stellung arbeitet die Lichtapplikationseinheit 10 insbesondere für die photodynamische Diagnose.

An der Außenseite, in der Darstellung von Fig. 1 an der Unterseite, ist über eine Halterung 44 ein drittes Filter 46 angeordnet. Über die Halterung 44 ist das dritte Filter 46 verschwenkbar und auch längs des Rohrabschnittes 16 verschiebbar angebracht.

Dieses dritte Filter 46 dient dazu, um zwischen das Auge 48 einer Beobachtungs- oder Handhabungsperson der Lichtapplikationseinheit 10 und dem durch den Lichtleiter 23 bestrahlten Bereich gebracht zu werden. Dieses dritte Filter 46 ist so ausgebildet, daß es das Anregungslicht, das durch das erste Filter 40 hindurchdringt und vom Lichtleiter 23 geleitet wird, abblockt, Fluoreszenzlicht jedoch transmittiert, wodurch eine Detektion der gewebecharakterisierenden Fluoreszenz des bestrahlten Bereiches möglich ist.

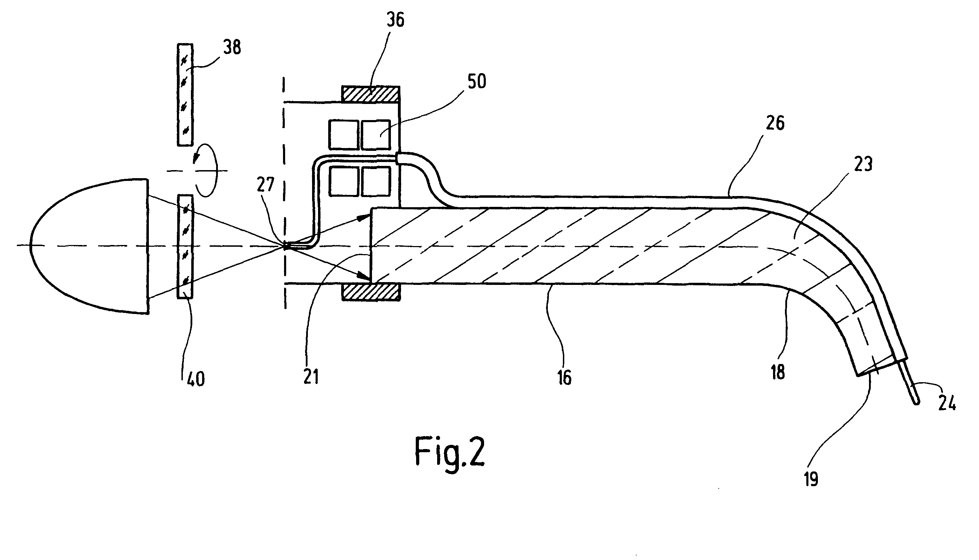

Bei der in Fig. 2 dargestellten Variante einer Lichtapplikationseinheit, bei der für gleiche Bauteile gleiche Bezugsziffern wie in Fig. 1 verwendet werden, ist im proximalen Endbereich des zweiten Lichtleiters 24 eine Ultraschallanregungseinheit 50 vorgesehen, die den Lichtleiter 24 in diesem Abschnitt umgibt. Der Lichtleiter 24 dient also in diesem Fall nicht nur dazu, das von der Lichtquelle 20 kommende Licht zu leiten, sondern auch um den eingekoppelten Ultraschall zum proximalen Ende des Lichtleiters 24 zu leiten. In diesem Fall besteht der zweite Lichtleiter 24 vorteilhafterweise aus Quarzglas.

Wie zuvor erwähnt, ist der Rohrabschnitt 16 über die Kupplung 36 durch einen einfachen Vorgang vom Gehäuse 12 abzunehmen bzw. an dieses anzukoppeln.

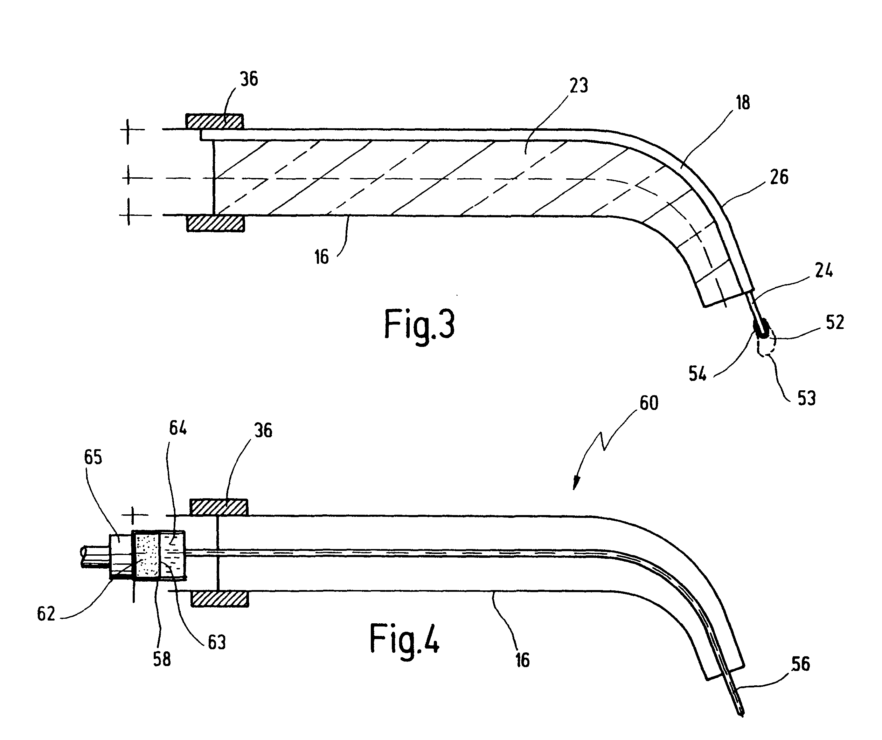

Bei der in Fig. 3 dargestellten Variante eines Rohrabschnittes 16 ist am äußeren distalen Ende des zweiten Lichtleiters 24 noch ein Diffusor 52 angeordnet.

Dieser Diffusor 52 dient dazu, das aus der relativ kleinen stirnseitigen Endfläche austretende Licht gleichmäßig zu verteilen, so daß über einen großen Bereich rundum gleichmäßig Licht abgestrahlt wird.

Ist der Diffusor 52 relativ steif ausgebildet, kann er auch zugleich noch als Abstandshalter 54 fungieren.

In einer Ausgestaltung ist vorgesehen, den Diffusor 52 als Ballon 53 auszubilden, der mit dem Kanal 26 in Verbindung steht, so daß über diesen ein Medium zugeführt werden kann, um den Ballon aufzublähen, um dann vor Ort eine relativ großflächige Abstrahlfläche zu erzeugen.

Bei der in Fig. 4 dargestellten Variante ist im Rohrabschnitt 16 mittig eine Kanüle 56 vorgesehen, die proximal mit einem Vorratsbehälter 58 verbunden ist.

Der Vorratsbehälter 58 weist eine erste Kammer 62 und eine zweite Kammer 64 auf. In der ersten Kammer 62 ist 5-Aminolävulinsäure oder eines ihrer Derivate, beispielsweise der Hexylester, und ein Alginatgel, jeweils in Pulverform, aufgenommen. In der zweiten Kammer 64 ist eine Trägersubstanz, beispielsweise eine Pufferlösung, enthalten. Ein Mechanismus 65 dient dazu, die in den beiden Kammern 62 und 64 enthaltenen Substanzen erst beim Applizieren miteinander zu vermischen und anschließend durch die Kanüle 56 auszutreiben. Die Pufferlösung stellt dabei einen pH 5-6 ein. Nach Vermischen stellt das Alginatgel eine Trägersubstanz dar.

Wird der in Fig. 4 dargestellte Rohrabschnitt 16 an das Gehäuse 12 angekoppelt, kann die Lichtapplikationseinheit 10 auch gleichzeitig als Vorrichtung 60 zum Applizieren des Photosensitizers eingesetzt werden.

Wird dies nicht gewünscht, kann diese Vorrichtung als separate Vorrichtung ausgebildet sein, über die das pharmazeutische Präparat appliziert wird. Dann besteht der Mechanismus 65 beispielsweise aus einem Stempel, der die Trennwand 63 zwischen den beiden Kammern 62 und 64 zerstört und deren Inhalte miteinander vermischt und dann über die Kanüle 56 austreibt.

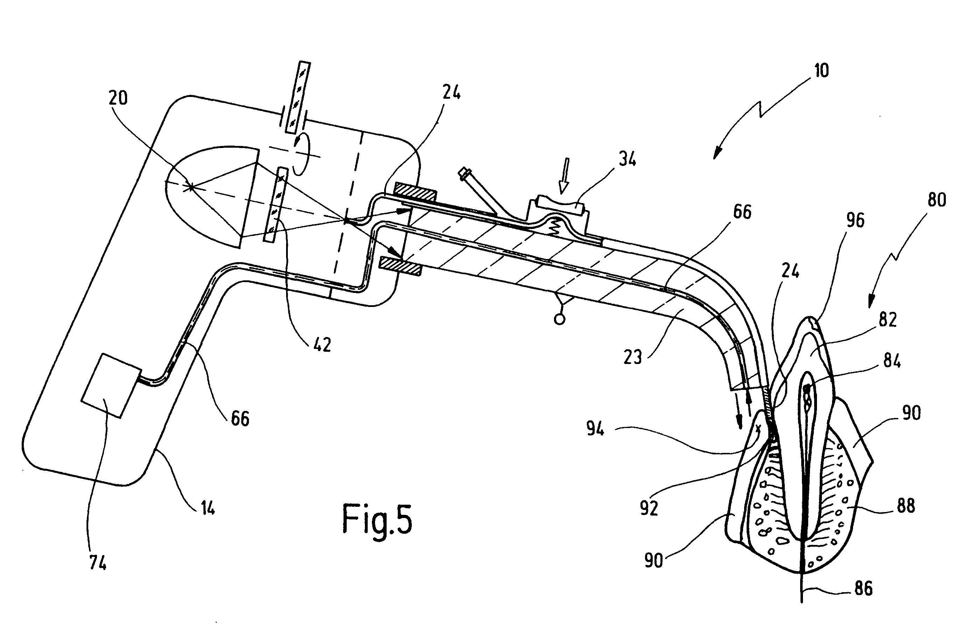

In Fig. 5 ist eine Anwendung einer Lichtapplikationseinheit 10 zur Therapie beschrieben, wobei hier eine Variante gezeigt ist, bei der der erste Lichtleiter 23 so ausgebildet ist, daß einige Lichtleitfasern dazu herangezogen werden, um Licht von distal nach proximal zu führen. Diese Lichtleiter 66 stehen mit einer Auswerteinheit 74 in Verbindung, die hier im Handgriff 14 angeordnet ist, die auch außerhalb angeordnet sein kann, so daß dann dementsprechend eine lichtleitende Verbindung vorgesehen ist.

In Fig. 5 ist ein Ausschnitt des menschlichen Zahnhalteapparates 80 dargestellt.

Dieser weist einen Zahn 82 auf, in dessen innerer Zahnhöhle 84 der Nerv 86 aufgenommen ist.

Das untere Ende des Zahnes 82 sitzt im Kieferknochen 88 und ist seitlich vom Zahnfleisch 90 umgeben.

Im Rahmen einer Erkrankung hat sich zwischen der Außenseite des Zahnes 82 und dem Zahnfleisch 90 eine Tasche 92 ausgebildet.

In diese Tasche 92 wurde zuvor eine Mischung aus 5-Aminolävulinsäurehexylester und Alginatgel appliziert und ca. zwei Stunden abgewartet. Das Alginatgel entstand aus dem Alginatpulver und der Pufferlösung. Durch die hohe Penetrationsgeschwindigkeit und die hohe Eindringtiefe des Esters ist zwischenzeitlich der Prekursor des Photosensitizers bis zu einem inneren Bereich 94 im Zahnfleisch gedrungen und hat dort in dem befallenen Bereich zu einer Erhöhung des Photosensitizers Protoporphyrin IX geführt.

Die Lichtapplikationseinheit 10 wird nun so an dem Zahnhalteapparat 80 angesetzt, daß der verschiebbare zweite Lichtleiter 24 bzw. dessen vom Rohrabschnitt 16 vorstehendes distales Ende in die Tasche 92 eingeschoben wird. Durch die Verschiebbarkeit wird dieser Vorgang erleichtert.

Der erste Lichtleiter 23 führt Licht an die Außenseite des Zahnfleisches 90. Anschließend wird die Lichtquelle 20, eine vorzugsweise im Blauen strahlende Xenon-Kurzbogenlampe, aktiviert. Das zweite Filter 42 transmittiert im Bereich des Anregungsspektrums des Photosensitizers, und dieses Licht wird sowohl über den ersten Lichtleiter 23 als auch über den zweiten Lichtleiter 24 an den Zahnhalteapparat 80 herangeführt. Durch die beidseitige Bestrahlung wird der innere Bereich 94 optimal therapiert. Einige Lichtleiter 66 des ersten Lichtleiters 23 dienen dazu, Fluoreszenzlicht, das vom Photosensitizer abgestrahlt wird, von distal nach proximal zu führen. Diese Lichtleiter 66 stehen mit einer Auswerteeinheit 74 in Verbindung.

Diese Auswerteeinheit 74 detektiert im Laufe der Therapie die Abnahme der Fluoreszenzstrahlung und somit den Verlauf der Zerstörung des befallenen Gewebes durch die photodynamische Therapie. Zum Ausfiltern von ebenfalls zurückleitendem Anregungslicht sind Filter vorgesehen, die nur Fluoreszenzlicht transmittieren.

In Fig. 5 ist eine durch Karies befallene Stelle des Zahnes 82 mit der Bezugsziffer 96 versehen. Es ist ersichtlich, daß auch diese Stelle 96 mittels der Lichtapplikationseinheit 10 sowohl diagnostiziert als auch therapiert werden kann. Die beschriebene Lichtapplikationseinheit ist prinzipiell auch für andere Photosensitizer einsetzbar, die dasselbe Phänomen am Zahnhalteapparat bzw. an den Zähnen zeigen.

## Patentansprüche

1. Lichtapplikationseinheit zur kombinierten photodynamischen Diagnose und/oder photodynamischen Therapie von nicht-malignen Erkrankungen des Zahnhalteapparates (80) und der Zähne (82), mit einer Lichtquelle (20), die zumindest Licht im sichtbaren Bereich erzeugt, mit einer Fokussiereinheit (22) zum Fokussieren des Lichtes, mit zumindest einem Lichtleiter (23, 24), der Licht von der Lichtquelle (20) zu einem distalen, abstrahlenden Ende (19, 25) der Lichtleiter (23, 24) führt, mit zumindest einem im Strahlengang des Lichts anordenbaren Element (38), mit dem die spektralen Eigenschaften des Lichtes der Lichtquelle (20) veränderbar sind, wobei der Lichtleiter (23, 24) zumindest in einem distalen Handhabungsbereich rigide ausgebildet ist und zwei Lichtleiter (23, 24) vorgesehen sind, **dadurch gekennzeichnet, daß** die Lichtleiter (23, 24) distalseitig einen gekrümmten Abschnitt aufweisen und daß die beiden Lichtleiter (23, 24) nebeneinander verlaufen, wobei eine abstrahlseitige Stirnfläche des einen Lichtleiters (24) über die abstrahlseitige Stirnfläche des anderen Lichtleiters (23) distalseitig hinausragt.

2. Lichtapplikationseinheit nach Anspruch 1, **dadurch gekennzeichnet, daß** einer der Lichtleiter (24) relativ axial verschiebbar zum anderen Lichtleiter (23) angeordnet ist.

3. Lichtapplikationseinheit nach Anspruch 2, **dadurch gekennzeichnet, daß** der verschiebliche Lichtleiter (24) in einem rohrförmigen Kanal (26) geführt ist.

4. Lichtapplikationseinheit nach Anspruch 3, **dadurch gekennzeichnet, daß** der rohrförmige Kanal (26) zur Führung von Medien ausgebildet ist.

5. Lichtapplikationseinheit nach einem der Ansprüche 1 bis 4, **dadurch gekennzeichnet, daß** das im Strahlengang anordenbare Element (38) als Filter (40, 42) ausgebildet ist.

6. Lichtapplikationseinheit nach Anspruch 5, **dadurch gekennzeichnet, daß** unterschiedliche Filter (40, 42) in den Strahlengang ein- bzw. ausbringbar angeordnet sind.

7. Lichtapplikationseinheit nach einem der Ansprüche 1 bis 6, **dadurch gekennzeichnet, daß** ein drittes Filter (46) vorgesehen ist, das in eine Blickrichtung von einer Handhabungsperson zu einer Behandlungsstelle am Zahnhalteapparat (80) bringbar ist, wobei dieses dritte Filter Fluoreszenzanregungslicht abblockt, jedoch Fluoreszenzstrahlung durchläßt.

8. Lichtapplikationseinheit nach einem der Ansprüche 1 bis 7, **dadurch gekennzeichnet, daß** ein Lichtleiter (23, 24) aus einer Vielzahl von lichtleitenden Einzelfasern zusammengesetzt ist.

9. Lichtapplikationseinheit nach einem der Ansprüche 1 bis 7, **dadurch gekennzeichnet, daß** ein Lichtleiter aus einer einzigen lichtleitenden Faser aufgebaut ist.

10. Lichtapplikationseinheit nach einem der Ansprüche 1 bis 9, **dadurch gekennzeichnet, daß** ein weiterer Lichtleiter (66) vorgesehen ist, der Licht von der bestrahlten Stelle nach proximal leitet.

11. Lichtapplikationseinheit nach Anspruch 10, **dadurch gekennzeichnet, daß** der weitere Lichtleiter (66) Teil eines mehrfaserigen Lichtleiters (23) ist, der an sich Licht nach distal leitet.

12. Lichtapplikationseinheit nach Anspruch 10 oder 11, **dadurch gekennzeichnet, daß** das vom weiteren Lichtleiter (66) nach proximal geleitete Licht einer Auswerteeinheit (74) zugeführt wird.

13. Lichtapplikationseinheit nach Anspruch 12, **dadurch gekennzeichnet, daß** die Auswerteeinheit (74) eine Spektralanalyse-Einheit, eine Kamera oder ein spektral selektiv sensitives Photoelement aufweist.

14. Lichtapplikationseinheit nach einem der Ansprüche 1 bis 13, **dadurch gekennzeichnet, daß** der Lichtleiter (24) distalseitig mit einem Abstandshalter (54) versehen ist.

15. Lichtapplikationseinheit nach einem der Ansprüche 1 bis 14, **dadurch gekennzeichnet, daß** der Lichtleiter (24) distalseitig einen Diffusor (52) aufweist.

16. Lichtapplikationseinheit nach Anspruch 15, **dadurch gekennzeichnet, daß** der Diffusor (52) als kugelförmig konvexe Aufweitung des distalen Endes des Lichtleiters (24) ausgebildet ist.

17. Lichtapplikationseinheit nach Anspruch 15 oder Anspruch -16, **dadurch gekennzeichnet, daß** der Diffusor (52) als lichtdurchlässiges Material mit optisch streuenden Eigenschaften ausgebildet ist.

18. Lichtapplikationseinheit nach einem der Ansprüche 15 bis 17, **dadurch gekennzeichnet, daß** der Diffusor (52) als füllbarer Ballon (53) ausgebildet ist, der am distalen Ende des Lichtleiters (24) angeordnet ist und über einen Kanal (26) füllbar ist.

19. Lichtapplikationseinheit nach einem der Ansprüche 1 bis 18, **dadurch gekennzeichnet, daß** ein Ultraschallübertragungselement vorgesehen ist, über das Ultraschall zum distalen Ende leitbar ist.

20. Lichtapplikationseinheit nach Anspruch 19, **dadurch gekennzeichnet, daß** eine Ultraschallanregungseinheit (50) vorgesehen ist, die mit dem Ultraschallübertragungselement gekoppelt ist.

21. Lichtapplikationseinheit nach Anspruch 19 oder 20, **dadurch gekennzeichnet, daß** ein Lichtleiter (24) als Ultraschallübertragungselement ausgebildet ist.

22. Lichtapplikationseinheit nach einem der Ansprüche 1 bis 21, **dadurch gekennzeichnet, daß** die Lichtquelle (20) einen hohen Blauanteil im Bereich um 400 nm aufweist.

23. Lichtapplikationseinheit nach Anspruch 22, **dadurch gekennzeichnet, daß** die Lichtquelle (20) eine Xenon-Kurzbogenlampe aufweist.

24. Lichtapplikationseinheit nach einem der Ansprüche 1 bis 23, **dadurch gekennzeichnet, daß** sie ein Gehäuse (12) mit einem pistolenartigen Handgriff (14) aufweist, von dem ein Rohrabschnitt (16) vorsteht, in dem die Lichtleiter (23, 24) geführt sind.

25. Lichtapplikationseinheit nach Anspruch 24, **dadurch gekennzeichnet, daß** der Rohrabschnitt (16) an das Gehäuse (12) ankoppelbar bzw. von diesem abnehmbar ausgebildet ist.

26. Lichtapplikationseinheit nach Anspruch 24 oder 25, **dadurch gekennzeichnet, daß** der Lichtleiter (23) und der Rohrabschnitt (16) zumindest über einen Teil deren Länge zum distalen Ende (19) hin konisch verjüngend ausgebildet sind.

27. Lichtapplikationseinheit nach einem der Ansprüche 7 bis 26, **dadurch gekennzeichnet, daß** das dritte Filter (46) am Rohrabschnitt (16) angebracht ist.

28. Lichtapplikationseinheit nach Anspruch 27, **dadurch gekennzeichnet, daß** das dritte Filter (26) verschieblich und verschwenkbar am Rohrabschnitt (16) angebracht ist.

29. Lichtapplikationseinheit nach einem der Ansprüche 1 bis 28, **dadurch gekennzeichnet, daß** eine Kanüle (56) vorgesehen ist, über die das pharmazeutische Präparat applizierbar ist.

## Revendications

1. Unité d'application de lumière pour le diagnostic et/ou la thérapie photodynamiques combinés d'affections non malignes du parodonte (80) et des dents (82), comportant une source de lumière (20) qui produit au moins de la lumière dans le domaine visible, comportant une unité de focalisation (22) pour focaliser la lumière, comportant au moins un guide optique (23, 24) qui guide la lumière de la source de lumière (20) à une extrémité distale émettrice (19, 25) des guides optiques (23, 24), comportant au moins un élément (38) pouvant être disposé dans le parcours des rayons de la lumière, au moyen duquel on peut modifier les propriétés spectrales de la lumière de la source de lumière (20), le guide optique (23, 24) étant réalisé rigide au moins dans une zone de manipulation distale et deux guides optiques (23, 24) étant prévus, **caractérisée en ce que** les guides optiques (23, 24) comportent côté distal un tronçon courbé et **en ce que** les deux guides optiques (23, 24) s'étendent côte à côte, une face frontale côté émission d'un guide optique (24) dépassant côté distal de la face frontale côté émission de l'autre guide optique (23).

2. Unité d'application de lumière selon la revendication 1, **caractérisée en ce que** l'un des guides optiques (24) est disposé de manière à pouvoir coulisser axialement par rapport à l'autre guide optique (23).

3. Unité d'application de lumière selon la revendication 2, **caractérisée en ce que** le guide optique (24) coulissant est guidé dans un canal (26) de forme tubulaire.

4. Unité d'application de lumière selon la revendication 3, **caractérisée en ce que** le canal (26) de forme tubulaire est réalisé pour guider des fluides.

5. Unité d'application de lumière selon l'une des revendications 1 à 4, **caractérisée en ce que** l'élément (38), pouvant être disposé dans le parcours des rayons, est réalisé sous forme de filtre (40, 42).

6. Unité d'application de lumière selon la revendication 5, **caractérisée en ce que** différents filtres (40, 42) sont disposés de manière à pouvoir être introduits ou extraits du parcours des rayons.

7. Unité d'application de lumière selon l'une des revendications 1 à 6, **caractérisée en ce qu'**il est prévu un troisième filtre (46) qui peut être amené sur le parodonte (80), dans le sens d'observation depuis un opérateur jusqu'à un emplacement de traitement, ce troisième filtre bloquant la lumière d'excitation fluorescente, mais laissant passer le rayonnement fluorescent.

8. Unité d'application de lumière selon l'une des revendications 1 à 7, **caractérisée en ce qu'**un guide optique (23, 24) se compose d'un grand nombre de fibres individuelles guidant la lumière.

9. Unité d'application de lumière selon l'une des revendications 1 à 7, **caractérisée en ce qu'**un guide optique est constitué d'une seule fibre guidant la lumière.

10. Unité d'application de lumière selon l'une des revendications 1 à 9, **caractérisée en ce qu'**il est prévu un autre guide optique (66) qui guide la lumière depuis l'emplacement irradié vers le côté proximal.

11. Unité d'application de lumière selon la revendication 10, **caractérisée en ce que** l'autre guide optique (66) fait partie d'un guide optique (23) à plusieurs fibres qui guide la lumière vers le côté distal.

12. Unité d'application de lumière selon la revendication 10 ou 11, **caractérisée en ce que** la lumière guidée par l'autre guide optique (66) vers le côté proximal est amenée à une unité d'exploitation (74).

13. Unité d'application de lumière selon la revendication 12, **caractérisée en ce que** l'unité d'exploitation (74) comporte une unité d'analyse spectrale, une caméra ou un photo-élément sensible de manière sélective aux spectres.

14. Unité d'application de lumière selon l'une des revendications 1 à 1 3, **caractérisée en ce que** le guide optique (24) est pourvu d'un écarteur (54) côté distal.

15. Unité d'application de lumière selon l'une des revendications 1 à 14, **caractérisée en ce que** le guide optique (24) comporte un diffuseur (52) côté distal.

16. Unité d'application de lumière selon la revendication 15, **caractérisée en ce que** le diffuseur (52) est réalisé comme élargissement convexe en forme de sphère de l'extrémité distale du guide optique (24).

17. Unité d'application de lumière selon la revendication 15 ou la revendication 16, **caractérisée en ce que** le diffuseur (52) est réalisé sous la forme d'un matériau perméable à la lumière avec des propriétés de diffusion optique.

18. Unité d'application de lumière selon l'une des revendications 15 à 17, **caractérisée en ce que** le diffuseur (52) est réalisé sous la forme d'un ballon (53) pouvant être rempli, qui est disposé à l'extrémité distale du guide optique (24) et peut être rempli par un canal (26).

19. Unité d'application de lumière selon l'une des revendications 1 à 18, **caractérisée en ce qu'**il est prévu un élément de transmission des ultrasons par lequel des ultrasons peuvent être guidés jusqu'à l'extrémité distale.

20. Unité d'application de lumière selon la revendication 19, **caractérisée en ce qu'**il est prévu une unité d'excitation des ultrasons (50) qui est couplée à l'élément de transmission des ultrasons.

21. Unité d'application de lumière selon la revendication 19 ou 20, **caractérisée en ce qu'**un guide optique (24) est réalisé comme élément de transmission des ultrasons.

22. Unité d'application de lumière selon l'une des revendications 1 à 21, **caractérisée en ce que** la source de lumière (20) présente une composante bleue importante de l'ordre de 400 nm.

23. Unité d'application de lumière selon la revendication 22, **caractérisée en ce que** la source de lumière (20) comporte une lampe au xénon à arc court.

24. Unité d'application de lumière selon l'une des revendications 1 à 23, **caractérisée en ce qu'**elle comporte un boîtier (12) avec une poignée (14) de type pistolet de laquelle dépasse un tronçon de tube (16) dans lequel sont guidés les guides optiques (23, 24).

25. Unité d'application de lumière selon la revendication 24, **caractérisée en ce que** le tronçon de tube (16) est réalisé de manière à pouvoir être accouplé au boîtier (12) ou retiré de celui-ci.

26. Unité d'application de lumière selon la revendication 24 ou 25, **caractérisée en ce que** le guide optique (23) et le tronçon de tube (16) sont réalisés de manière à se rétrécir coniquement vers l'extrémité distale (19), au moins sur une partie de leur longueur.

27. Unité d'application de lumière selon l'une des revendications 7 à 26, **caractérisée en ce que** le troisième filtre (46) est placé sur le tronçon de tube (16).

28. Unité d'application de lumière selon la revendication 27, **caractérisée en ce que** le troisième filtre (26) est placé coulissant et pivotant sur le tronçon de tube (16).

29. Unité d'application de lumière selon l'une des revendications 1 à 28, **caractérisée en ce qu'**il est prévu une canule (56) par laquelle on peut appliquer la préparation pharmaceutique.

## Claims

1. Light application unit for combined photodynamic diagnosis and/or photodynamic therapy of non-malignant diseases of the parodontium (80) and the teeth (82), comprising a light source (20) for generating light at least in the visible region, a focusing unit (22) for focusing the light and at least one wave guide (23, 24) for transmitting light from the light source (20) to a distal, emitting end (19, 25) of the wave guide (23, 24), wherein at least one element (38) is arrangeable in the light beam path with which the spectral properties of the light of the light source (20) can be altered, and wherein the wave guide (23, 24) is rigidly configured at least in a distal handling region and wherein two wave guides (23, 24) are provided, **characterized in that** the wave guides (23, 24) have a curved section at the distal side and **in that** the two wave guides (23, 24) run adjacent to one another and wherein an emitting end face of one wave guide (24) projects in the distal direction beyond the emitting end face of the other wave guide (23).

2. Light application unit of claim 1, **characterized in that** one of the wave guides (24) is arranged to be axially shiftable relative to the other wave guide (23).

3. Light application unit of claim 2, **characterized in that** the shiftable wave guide (24) is disposed in a tubular channel (26).

4. Light application unit of claim 3, **characterized in that** the tubular channel (26) is configured to supply media.

5. Light application unit of anyone of claims 1 through 4, **characterized in that** the element (38) arrangeable in the light beam path is configured as a filter (40, 42).

6. Light application unit of claim 5, **characterized in that** different filters (40, 42) are arranged to be brought into or out of the light beam path.

7. Light application unit of anyone of claims 1 through 6, **characterized in that** a third filter (46) is provided which can be disposed in a view direction of an operator to a treatment area of the parodontium (80), wherein the third filter blocks fluorescence excitation light, however transmits fluorescence emission.

8. Light application unit of anyone of claims 1 through 7, **characterized in that** one wave guide (23, 24) is composed of a plurality of light transmitting individual fibers.

9. Light application unit of anyone of claims 1 through 7, **characterized in that** one wave guide is formed of a single light transmitting fiber.

10. Light application unit of anyone of claims 1 through 9, **characterized in that** a further wave guide (66) is provided, which transmits light from a proximal direction from the irradiated region.

11. Light application unit of claim 10, **characterized in that** the further wave guide (66) is part of a multi-fiber wave guide (23) which transmits light in the distal direction.

12. Light application unit of claims 10 or 11, **characterized in that** the light transmitted proximally from the further wave guide (66) is supplied to an evaluation unit (74).

13. Light application unit of claim 12, **characterized in that** the evaluation unit (74) comprises a spectral analysis unit, a camera, or a spectrally selective sensitive photo element.

14. Light application unit of anyone of claims 1 through 13, **characterized in that** the wave guide (24) is provided with a spacer (54) at the distal end.

15. Light application unit of anyone of claims 1 through 14, **characterized in that** the wave guide (24) comprises a diffuser (52) at the distal end.

16. Light application unit of claim 15, **characterized in that** the diffuser (52) is configured as a spherical convex expansion of the distal end of the wave guide (24).

17. Light application unit of claim 15 or of claim 16, **characterized in that** the diffuser (52) is configured as light-permeable material with optically controllable properties.

18. Light application unit of anyone of claims 15 through 17, **characterized in that** the diffuser (52) is formed as an inflatable balloon (53), arranged at the distal end of the wave guide (24), which is inflatable via a channel (26).

19. Light application unit of anyone of claims 1 through 18, **characterized in that** an ultrasound transmission element is provided through which ultrasound can be transmitted to the distal end.

20. Light application unit of claim 19, **characterized in that** an ultrasound excitation unit (50) is provided to be coupled with the ultrasound transmission element.

21. Light application unit of claims 19 or 20, **characterized in that** one wave guide (24) is configured as the ultrasound transmission element.

22. Light application unit of anyone of claims 1 through 21, **characterized in that** the light source (20) has a large blue component in the region of 400 nm.

23. Light application unit of claim 22, **characterized in that** the light source (20) comprises a xenon discharge lamp.

24. Light application unit of anyone of claims 1 through 23, **characterized in that** it comprises a housing (12) with a pistol-like handle (14) from which a tube section (16) extends, in which the wave guides (23, 24) are disposed.

25. Light application unit of claim 24, **characterized in that** the tube section (16) is configured to be coupled to the housing (12) and can be removed thereon.

26. Light application unit of claims 24 or 25, **characterized in that** the wave guide (23) and the tube section (16) are formed to taper conically over at least one portion of their lengths toward the distal end (19).

27. Light application unit of anyone of claims 7 through 26, **characterized in that** the third filter (46) is mounted to the tube section (16).

28. Light application unit of claim 27, **characterized in that** the third filter (26) is mounted to the tube section (16) to be shiftable and pivotable.

29. Light application unit of anyone of claims 1 through 28, **characterized in that** a canula (56) is provided through which the pharmaceutical preparation can be administered.
